# EUROPEAN PATENT SPECIFICATION

(11) **EP 0 724 458 B1**
(45) Date of publication and mention of the grant of the patent: **25.11.1998**
(21) Application number: 94924975.9
(22) Date of filing: 09.09.1994
(51) Int. Cl.: A61L 27/00, C22C 19/07, C23C 14/48

(54) **NOVEL COBALT CHROME ALLOYS AND PREPARATION**
NEUE KOBALT-CHROMLEGIERUNGEN UND HERSTELLUNG
NOUVEAUX ALLIAGES COBALT-CHROME ET LEUR PREPARATION

(30) Priority: 27.10.1993 US 144950
(43) Date of publication of application: 07.08.1996
(73) Proprietor: HOWMEDICA INC., New York New York 10017 (US)
(72) Inventor: HIGHAM, Paul, A., Ringwood, NJ 07456 (US); WARFIELD, Larry, T., Hellertown, PA 18055 (US)
(74) Representative: Ruddock, Keith Stephen
(86) International application number: IB9400271
(87) International publication number: WO9511708

(56) References cited:
- EP-A- 0 555 033
- WO-A-91/16013
- US-A- 4 693 760

## Description

This invention relates to new alloys of cobalt chromium (CoCr) (also often referred to herein as "cobalt chrome") and their preparation, and it relates in particular to new alloys of cobalt chrome having improved properties and being suitable for use against ultra high molecular weight polyethylene (i.e., UHMWPE) surfaces in opposing friction couples (for example in knees and hips in the field of orthopedics).

In the prior art, pure ceramic zirconia femoral heads for use in artificial hips are commercially available. These devices have some disadvantages, however. For example, the materials are expensive and they are more susceptible to fracture than metal heads.

Also known in the prior art are zirconium alloy femoral heads. These are made, for example, by Smith-Nephew Company. These also have the disadvantage that they are very expensive.

It is also known to implant nitrogen ions into cobalt chrome and into titanium alloys for applications in the field of orthopedics. However, nitrogen implantation does not produce good wear properties for titanium alloys against UHMWPE. See for example H.A. McKellup, T.V. Rostlund, J. of Biomedical Materials Research, vol. 24, pp. 1413-1425 (1990).

Therefore, new materials with improved properties were sought, especially, for orthopedic applications.

An object of this invention is a new material with improved properties to be used against ultra high molecular weight polyethylene (UHMWPE) surfaces in opposing friction couples (for example in knees and hips).

Another object of this invention is a method of making a cobalt chrome alloy that exhibits more ceramic-like properties than have been previously obtainable in such alloys.

Yet another object of this invention is a new and improved cobalt chrome alloy and articles of manufacture (e.g. knee replacements and femoral heads) prepared therefrom.

### Summary of the Invention

These and other objects are satisfied by the method of the invention of producing a new cobalt chrome alloy comprising: (a) implanting a substrate of cobalt chrome alloy with zirconium ions (preferably from a pure zirconium target) so as to produce a new cobalt chrome alloy implanted with zirconium.

Additionally and optionally, the new zirconium implanted cobalt chrome alloy can be coated (if desired) with a coating of zirconium.

When the cobalt chrome alloy is in the form of an orthopedic device, the method further comprises subjecting the device to sterilization procedures for example by gamma irradiation, which are known in the art.

Also according to the invention, a new cobalt chrome alloy is provided comprising cobalt chrome which has been treated by implanting the surface of the cobalt chrome with zirconium. This alloyed surface is then permitted to oxidize through contact with ambient air.

It is believed that zirconium ions have not previously been implanted into any material and in particular not into cobalt chrome alloy.

It has been found that a very thin gradient of zirconium metal adds life to the part which is subjected to wear, in spite of the shallow depth of implantation with zirconium. Additionally, the cost of manufacture is minimized by reducing the quantity of zirconium needed to effect the observed improvements.

Unexpectedly, it has been found that a very desirable combination of properties are obtained for the cobalt chrome alloy which has been implanted with zirconium ions as compared with the cobalt chrome alloy not subjected to implantation by zirconium. These properties include improved resistance to abrasion of the cobalt chrome alloy by bone cement; improved resistance of the alloy to corrosion by saline; and reduced wear of the alloy when articulated against the surface of UHMWPE.

### Description of the Preferred Embodiments

### Brief Description of the Drawings

Figures 1-3 are plots of applied potential versus current for zirconium implanted CoCr alloy (see Example 1 described below), untreated CoCr (Vitallium)(see Example 1 described below), and zirconium coated CoCr alloy (see Example 2 described below), respectively. (See Example 1, described below).

Fig. 4 is a plot of total weight loss of UHMWPE vs. number of complete cycles in a reciprocating wear test for zirconium implanted CoCr (described below in Example 1).

In the practice of the invention, when the new material is to be used for subsequent orthopedic purposes, any cobalt chrome alloy can be used as the substrate to be implanted with zirconium, provided that it is manufactured under the procedures of ASTM standard F75, which describes the standard procedures for producing orthopedic cobalt chrome alloys. One such suitable cobalt chrome alloy is produced by Howmedica Inc. and is sold under the trade name of Vitallium®.

The ASTM standard procedure F75 for the preparation of cobalt chrome alloy is hereby incorporated herein by reference.

The zirconium ions are implanted into the cobalt chrome alloy by the following procedure. Pure zirconium is used as the target. The general procedure for performing ion implantation into a substrate is described in the article by James R. Treglio, et al., "Performance of the Advanced MEVVA® IV 8-10 Metal Ion Implantation System", Surface and Coatings Technology, 51, (1992), 546-550. That article is hereby incorporated herein by reference. The general procedure described in that article was used. However, any of a number of standard commercially available ion implantation technologies will result in zirconium implantation of the CoCr surface.

In the practice of the invention, when the cobalt chrome substrate is an orthopedic device (for example a hip or a knee or other article of manufacture), the article of manufacture (following the step of zirconium ion implantation) must be subjected to sterilization procedures (for example, by gamma irradiation).

### Examples

The following Examples demonstrate the zirconium modification of cobalt chrome alloys and associated experimental data.

### Example 1

### Zirconium Implantation of Cobalt Chromium (ASTM 799) Alloy

Ion implantation, using Zirconium as the implant species, was carried out using an MEVVA IV (Metal Vapor Vacuum Arc) ion source as a commercial source, by ISM Technologies, San Diego, California. The MEVVA ion source generates a plasma via a cathodic arc. Highly polished (Average roughness, Rₐ < 100 Å) Cobalt Chromium discs, one inch in diameter, and curved wear test specimens were placed in the chamber 90 cm from the ion source, aligned such that the center of the discs was at the approximate center of the beam. The vacuum chamber was pumped down to a base pressure of 3x10⁻⁷ Torr by an oil free roughing pump and a cryogenic pump. The combination results in an oil free system, precluding any hydrocarbon contamination of treated surfaces. Zirconium was implanted into the discs using an accelerating voltage of 70 kV to a minimum dose of 2x10¹⁷/cm². This produced a surface implanted with Zirconium metal atoms to a depth of approximately 800 Å. The implanted Cobalt Chromium discs were evaluated to determine their coefficient of friction in bovine serum, corrosion resistance in saline, universal vickers hardness, and abrasion resistance to bone cement.

### Coefficient of Friction

The coefficient of friction for the UHMWPE - Zirconium implanted CoCr wear couple was determined using a commercially available pin-on-disc tribometer manufactured by Implant Sciences of Wakefield, MA. Conditions and the results of this testing are listed in Table 1A.

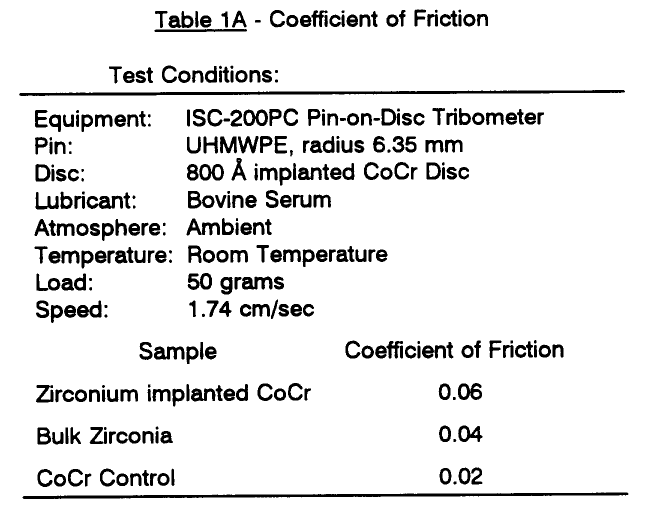

The implantation of CoCr with Zirconium has resulted in a coefficient of friction more like that of bulk Zirconia than that of cobalt chrome.

### Corrosion Resistance

The resistance of the Zr implanted CoCr to accelerated corrosion in saline was evaluated using the anodic polarization technique. A fixed area (1 cm²) of the disc was placed in saline, and current flow was monitored while increasing applied potential. Table 1B lists the test conditions and results.

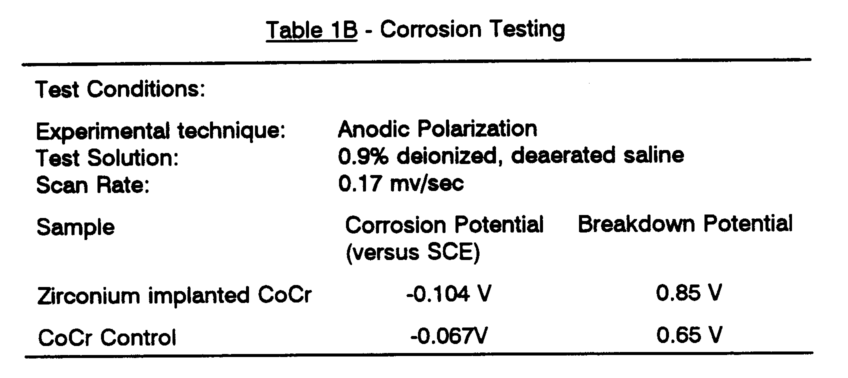

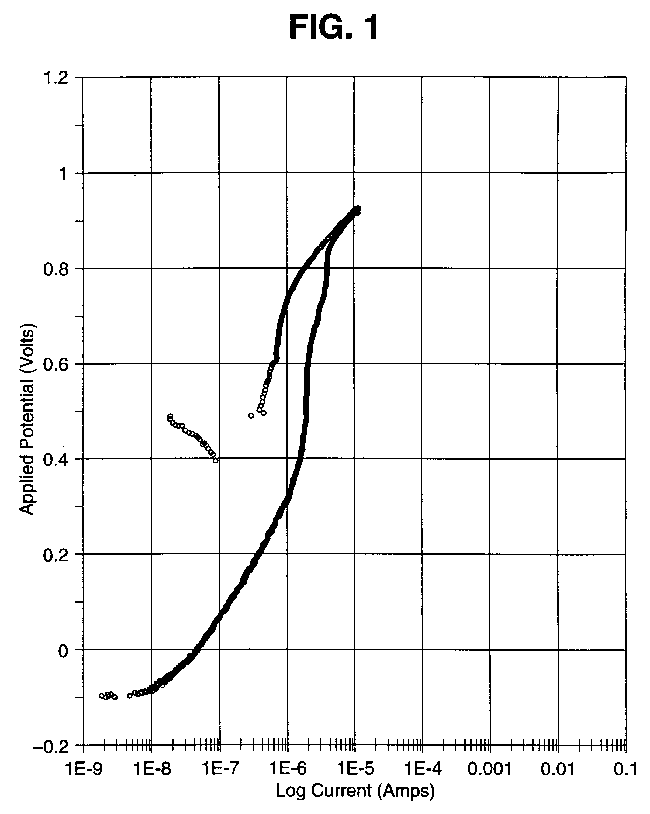

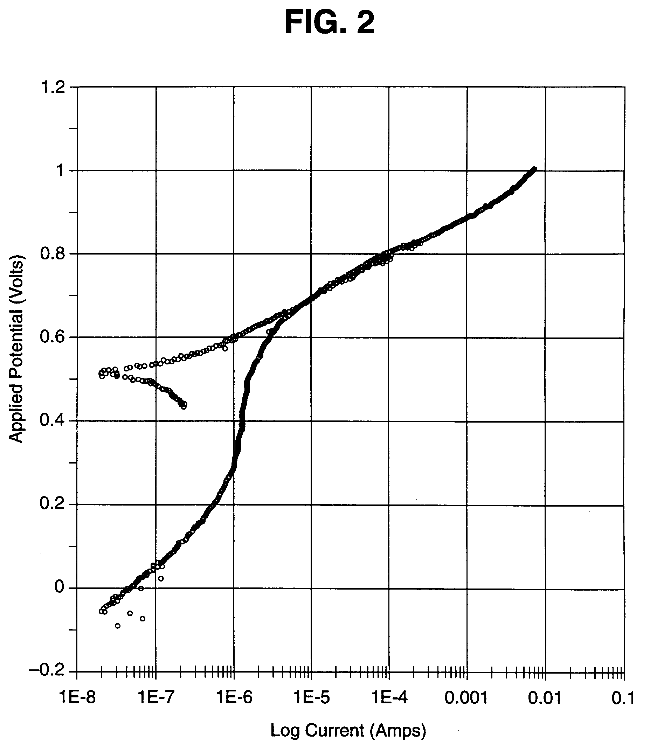

The Zirconium implanted CoCr showed a corrosion potential lower than the CoCr control. Additionally, the Zirconium implanted CoCr had a higher resistance to breakdown of the surfaces protective film than the CoCr control. When the potential was reversed, the Zirconium implanted CoCr sample showed immediate repair of the protective film, similar to the control, as indicated by an immediate decrease in current flow. (See Figures 1 and 2).

The results of this test indicate the Zirconium implanted CoCr has improved corrosion resistance over the untreated CoCr.

### Hardness

Using a Fischerscope H100V microhardness tester, the universal hardness values were determined for both the Zr treated and untreated CoCr. The Zirconium implanted sample had an ISE corrected hardness of 5936 ± 588 N/mm². Untreated wrought CoCr had a hardness of 5481 ± 188 N/mm². Thus the implantation did not result in significant hardening of the surface; however, this technique may be insensitive to such a shallow implantation depth. Thus the performance of the implanted material during wear and abrasion testing cannot be a result of increased surface hardness.

### Abrasion Testing

The Zirconium implanted CoCr discs were evaluated for their resistance to abrasion by Simplex bone cement. The flat discs were abraded using a hemispherical bone cement pin reciprocating against the disc under an applied load of approximately 1.25 Kg using water as a lubricant. The discs were checked for any resulting weight loss and any increased surface roughness. The pins were similarly checked for weight loss. The average weight loss for two treated discs was 0.22 mg, compared to 0.560 mg for an untreated control. The surface scar resulting from the abrading pin against the polished flat was much less for the Zr treated sample than for the untreated control. The Zr treated sample had a scar an average of 300 Å deep, compared to a scar of 1,980 Å for the untreated sample. Pins abrading against the Zr treated samples showed a weight loss of 4.9 mg, compared to 6.2 mg for the untreated controls. Thus the Zr implanted CoCr discs demonstrated significantly increased resistance to abrasion by bone cement, compared to untreated samples. This was not expected, based upon no significantly demonstrated improvements in hardness or coefficient of friction.

### Wear Testing

Forged Cobalt Chromium curved wear testing specimens were ion implanted to a depth of 800 Å with Zirconium as a commercial service by Implant Sciences, Wakefield, MA. The Zr implanted CoCr was loaded and reciprocated in 30% bovine serum against UHMWPE. The experimental conditions are listed in Table 1C.

**Table 1C**

| Wear Testing WEAR TEST CONDITIONS |
|---|
| EQUIPMENT: Reciprocating wear machine |
| LOAD: 250 lbs |
| FREQUENCY: 1 Hertz |
| OSCILLATING AMPLITUDE: ± 60 deg |
| DISTANCE PER CYCLE: 2.96 inches |
| LUBRICANT: 30% Bovine Serum diluted with Deionized water + 0.3% Sodium Azide |
| TEST COUNTER SURFACE: UHMWPE irradiated at 2.5 Mrads, presoaked for 4 weeks |

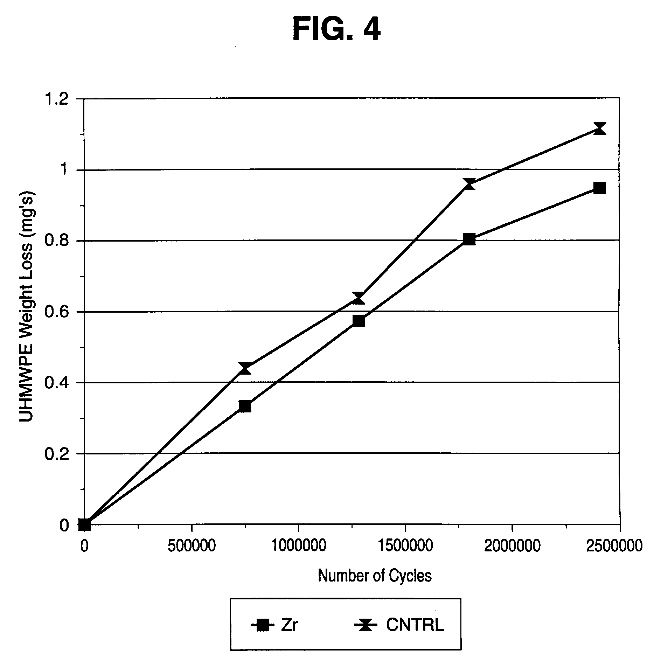

After 2.4 million cycles the weight losses of the UHMWPE counter-surfaces were measured. The UHMWPE test specimens articulating against the zirconium implanted sample lost only 0.953 mg, compared to an average of 1.12 mg for unimplanted CoCr controls. (See Figure 4) The untreated CoCr samples showed random scratches parallel to the wear direction, whereas the zirconia implanted CoCr specimens showed only minor scratches. Thus the zirconia implanted specimens reduced the wear of the UHMWPE counterfacing surface and exhibited improved resistance to abrasion.

### Example 2

Highly polished CoCr discs were ion implanted as per Example 1, with an additional deposition of a 2500 Å coating of Zirconium. These coatings were tested for corrosion and abrasion resistance, coefficient of friction, and universal hardness.

### Corrosion Resistance

The samples were tested using the identical conditions that were used in Example 1.

**Table 2**

| Corrosion Testing Anodic Polarization of CoCr coated with 2500 Å Zirconium | | |
|---|---|---|
| Sample | Corrosion Potential (Volts) | Breakdown Potential (Volts) |
| Zr Coated CoCr | -0.390 | 0.95 |
| CoCr Control | -0.067 | 0.65 |

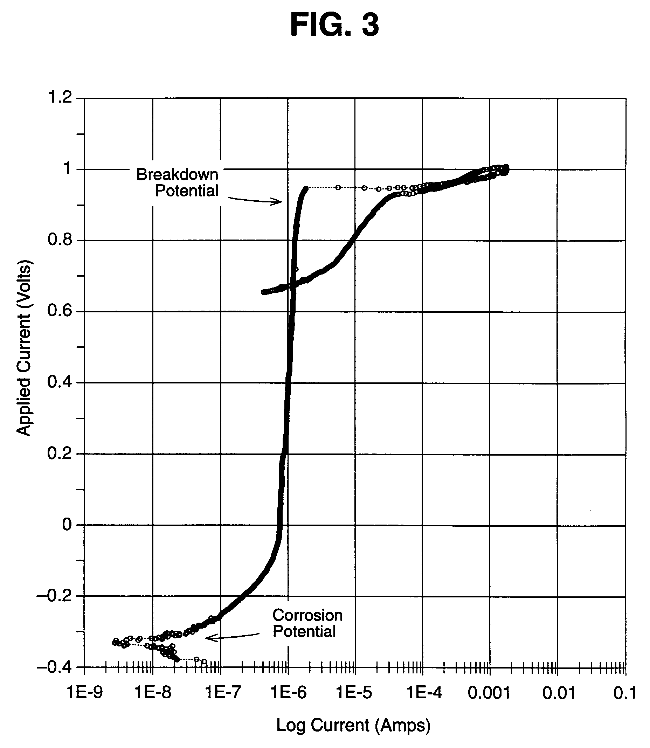

The Zirconium coated samples showed much lower corrosion potential and much higher breakdown potential than untreated CoCr (See Figure 3). This would indicate a significant improvement in resistance to corrosion in a saline environment.

### Hardness

Using a Fischerscope H100V microhardness tester, universal hardness values of 5303 ± 278 N/mm² were determined for the Zirconium coated samples. Untreated forged CoCr had a hardness of 5481 ± 188 N/mm². Bulk Zirconia, under identical test conditions had a hardness of 11709 ± 590 N/mm². Thus, the Zirconium coated samples probably reflect the hardness of the CoCr substrate because of the thinness of the surface coating.

### Coefficient of Friction

Under the same conditions as listed in Example 1, the Zirconium coated samples had a coefficient of friction of 0.12.

### Abrasion Resistance

Zirconium coated CoCr discs were tested for resistance to abrasion by bone cement under conditions identical to those listed in Example 1. After 1 million cycles, the Zirconium discs showed almost no visible scratching and had a weight loss of 0.06 mg (average), compared to 0.36 mg for untreated controls. This was an unexpected result since the coating shows a composite hardness (coating + substrate) less than the untreated CoCr and a coefficient of friction higher than the untreated CoCr control.

## Claims

1. A composition comprising a cobalt chrome alloy implanted with zirconium metal (not a as coating).

2. A composition according to claim 1 and including also a coating of zirconium.

3. A cobalt chrome alloy prepared by implanting a substrate of cobalt chrome alloy with zirconium ions from a pure zirconium target.

4. A composition according to claim 3, wherein said pure zirconium metal has been ionized, the ionized zirconium extracted and accelerated in order to impact the desired target with sufficient energy to implant zirconium into the surface of said cobalt chrome alloy.

5. A composition according to claim 4 wherein said surface has oxidized over a period of time.

6. A composition according to claim 5, and including also a coating thereon of zirconium.

7. A composition according to claim 3, wherein said pure zirconium is implanted in an oxidizing atmosphere so as to result in said zirconium being implanted as zirconia.

8. A composition according to claim 4, wherein said surface has been artificially oxidized to produce an oxide surface.

9. A composition according to claim 8, wherein said surface has been artificially oxidized by means selected from the group consisting of thermal means and chemical means.

10. A method of producing a cobalt chrome alloy, said method comprising implanting a substrate of cobalt chrome alloy with zirconium ions so as to produce said cobalt chrome alloy implanted with zirconium (not as a coating).

11. A method according to claim 10 wherein said zirconium ions for implanting said substrate of cobalt chrome alloy are obtained by ionizing a source of pure zirconium so as to produce zirconium ions, extracting and accelerating said zirconium ions towards a suitable cobalt chrome alloy substrate.

12. A method according to claim 11, wherein said cobalt chrome alloy implanted with zirconium is further subjected to a step of coating with zirconium.

13. A method according to claim 11, wherein said pure zirconium is implanted in an oxidizing atmosphere so as to result in said zirconium being implanted as zirconia.

14. A method according to claim 13, wherein said surface has been artificially oxidized to produce an oxide surface.

15. A method according to claim 14, wherein said surface has been artificially oxidized by means selected from the group consisting of thermal means and chemical means.

16. An article of manufacture comprising a cobalt chrome alloy implanted with zirconium ion (not as a coating).

17. An article according to claim 16, wherein said article is an orthopedic device.

18. An article according to claim 17 and including also a second part to be used together with said article of claim 17 in an opposing friction couple against said article of claim 17.

19. An article according to claim 18, wherein said second part comprises ultra-high molecular weight polyethylene.

20. An article according to claim 19, wherein said article is a knee implant.

21. An article according to claim 20, wherein said article is a femoral head.

## Patentansprüche

1. Zusammensetzung, umfassend eine Cobalt-Chrom-Legierung, implantiert mit Zirconiummetall (nicht als eine Beschichtung).

2. Zusammensetzung nach Anspruch 1, die ebenfalls eine Beschichtung aus Zirconium einschließt.

3. Cobalt-Chrom-Legierung, hergestellt durch Implantieren eines Substrats aus Cobalt-Chrom-Legierung mit Zirconiumionen aus einem reinen Zirconiumtarget.

4. Zusammensetzung nach Anspruch 3, wobei das reine Zirconiummetall ionisiert wurde, das ionisierte Zirconium extrahiert und beschleunigt wurde, um auf dem gewünschten Target mit ausreichender Energie aufzutreffen, damit Zirconium in die Oberfläche der Cobalt-Chrom-Legierung implantiert wird.

5. Zusammensetzung nach Anspruch 4, wobei die Oberfläche über einen gewissen Zeitraum oxidiert wurde.

6. Zusammensetzung nach Anspruch 5, die ebenfalls eine Beschichtung aus Zirconium darauf einschließt.

7. Zusammensetzung nach Anspruch 3, wobei das reine Zirconium in einer oxidierenden Atmosphäre implantiert wird, so daß das Zirconium als Zirconiumoxid implantiert ist.

8. Zusammensetzung nach Anspruch 4, wobei die Oberfläche zur Herstellung einer Oxidoberfläche künstlich oxidiert wurde.

9. Zusammensetzung nach Anspruch 8, wobei die Oberfläche mit Mitteln, ausgewählt aus der Gruppe, bestehend aus thermischen Mitteln und chemischen Mitteln, künstlich oxidiert wurde.

10. Verfahren zur Herstellung einer Cobalt-Chrom-Legierung, wobei das Verfahren Implantieren von Zirconiumionen in ein Substrat aus Cobalt-Chrom-Legierung umfaßt, unter Herstellung der mit Zirconium implantierten Cobalt-Chrom-Legierung (nicht als eine Beschichtung).

11. Verfahren nach Anspruch 10, wobei die Zirconiumionen zum Implantieren in das Substrat aus Cobalt-Chrom-Legierung durch Ionisieren einer Quelle aus reinem Zirconium, um Zirconiumionen herzustellen, Extrahieren und Beschleunigen der Zirconiumionen gegen ein geeignetes Cobalt-Chrom-Legierungssubstrat, erhalten werden.

12. Verfahren nach Anspruch 11, wobei die mit Zirconium implantierte Cobalt-Chrom-Legierung weiterhin einem Schritt der Beschichtung mit Zirconium unterzogen wird.

13. Verfahren nach Anspruch 11, wobei das reine Zirconium in einer oxidierenden Atmosphäre implantiert wird, so daß das Zirconium als Zirconiumoxid implantiert wird.

14. Verfahren nach Anspruch 13, wobei die Oberfläche zur Herstellung einer Oxidoberfläche - künstlich oxidiert wurde.

15. Verfahren nach Anspruch 14, wobei die Oberfläche mit Mitteln, ausgewählt aus der Gruppe, bestehend aus thermischen Mitteln und chemischen Mitteln, künstlich oxidiert wurde.

16. Herstellungsgegenstand, umfassend eine Cobalt-Chrom-Legierung, implantiert mit Zirconiumionen (nicht als eine Beschichtung).

17. Gegenstand nach Anspruch 16, wobei der Gegenstand eine orthopädische Vorrichtung ist.

18. Gegenstand nach Anspruch 17, der ebenfalls einen zweiten Teil einschließt, der zusammen mit dem Gegenstand von Anspruch 17 in einem gegenüberstehenden Reibungspaar gegen den Gegenstand von Anspruch 17 angewendet wird.

19. Gegenstand nach Anspruch 18, wobei der zweite Teil ein Polyethylen mit sehr hohem Molekulargewicht umfaßt.

20. Gegenstand nach Anspruch 19, wobei der Gegenstand ein Knieimplantat ist.

21. Gegenstand nach Anspruch 20, wobei der Gegenstand ein Oberschenkelknochenkopf ist.

## Revendications

1. Composition comprenant un alliage de cobalt et de chrome dans lequel est implanté du métal zirconium (pas en tant que revêtement).

2. Composition selon la revendication 1 et comprenant aussi un revêtement de zirconium.

3. Alliage de cobalt et de chrome préparé par implantation d'ions zirconium provenant d'une cible de zirconium pur dans un substrat formé d'un alliage de cobalt et de chrome.

4. Composition selon la revendication 3, dans laquelle ledit métal zirconium pur a été ionisé, on extrait et on accélère le zirconium ionisé de façon à lui faire heurter la cible désirée avec une énergie suffisante pour implanter le zirconium dans la surface dudit alliage de cobalt et de chrome.

5. Composition selon la revendication 4, ladite surface ayant été oxydée pendant un certain laps de temps.

6. Composition selon la revendication 5 et comprenant aussi un revêtement de zirconium à sa surface.

7. Composition selon la revendication 3, ledit zirconium pur étant implanté dans une atmosphère oxydante de façon à ce que ledit zirconium soit finalement implanté sous la forme de zircone.

8. Composition selon la revendication 4, ladite surface étant oxydée artificiellement pour produire une surface à base d'oxyde.

9. Composition selon la revendication 8, ladite surface étant oxydée artificiellement par un moyen choisi dans l'ensemble constitué par un moyen thermique et un moyen chimique.

10. Procédé de préparation d'un alliage de cobalt et de chrome, ledit procédé comprenant l'implantation d'ions zirconium dans un substrat en alliage de cobalt et de chrome de façon à produire ledit alliage de cobalt et de chrome dans lequel est implanté du zirconium (pas sous la forme d'un revêtement)

11. Procédé selon la revendication 10, dans lequel on obtient lesdits ions zirconium à implanter dans ledit substrat en alliage de cobalt et de chrome en ionisant une source de zirconium pur de façon à produire des ions zirconium, en extrayant et en accélérant lesdits ions zirconium vers un substrat approprié en alliage de cobalt et de chrome.

12. Procédé selon la revendication 11, dans lequel ledit alliage de cobalt et de chrome dans lequel est implanté du zirconium est soumis, en outre, à une étape de revêtement avec du zirconium.

13. Procédé selon la revendication 11, dans lequel ledit zirconium pur est implanté dans une atmosphère oxydante de façon à ce que le zirconium soit finalement implanté sous la forme de zircone.

14. Procédé selon la revendication 13, dans lequel on oxyde artificiellement ladite surface pour produire une surface à base d'oxyde.

15. Procédé selon la revendication 14, dans lequel on oxyde artificiellement ladite surface par un moyen choisi dans l'ensemble constitué par un moyen thermique et un moyen chimique.

16. Article manufacturé comprenant un alliage de cobalt et de chrome dans lequel des ions zirconium sont implantés (pas en tant que revêtement).

17. Article selon la revendication 16, ledit article étant un dispositif orthopédique.

18. Article selon la revendication 17 et comprenant aussi une seconde partie à utiliser conjointement avec ledit article de la revendication 17 dans un couple de frottement opposé par rapport audit article de la revendication 17.

19. Article selon la revendication 18, dans lequel la seconde partie comprend un polyéthylène de poids moléculaire très élevé.

20. Article selon la revendication 19, ledit article étant un implant pour le genou.

21. Article selon la revendication 20, ledit article étant une tête de fémur.
